# EUROPEAN PATENT APPLICATION

(11) **EP 2 514 412 A1**
(43) Date of publication of application: **24.10.2012**
(21) Application number: 12162963.8
(22) Date of filing: 30.04.2008
(51) Int. Cl.: A61K 9/19, A61K 47/34, A61K 38/28

(54) **Highly Concentrated Insulin Solutions and Compositions**

(30) Priority: 30.04.2007 EP 07107221; 08.06.2007 EP 07109906; 03.10.2007 EP 07117798
(62) Divisional of application: 08736644.9
(71) Applicant: NOVO NORDISK A/S, 2880 Bagsværd (DK)
(72) Inventor: Balschmidt, Per, DK-2880 Bagsvaerd (DK); Wahlund, Per-Oluf, DK-2880 Bagsvaerd (DK); Havelund, Svend, DK-2880 Bagsvaerd (DK); Bjerregaard Jensen, Simon, DK-2880 Bagsvaerd (DK)

(57) **Abstract**

Highly concentrated insulin solutions and pharmaceutical compositions are described as are methods of their preparation and uses thereof.

## Description

### FIELD OF THE INVENTION

One objective of the invention is to provide highly concentrated insulin solutions and compositions, methods of their preparation, and uses thereof. The invention thus provides highly concentrated insulin aqueous solutions suited for development of dosage forms suitable for several alternative routes of administration including, but not limited to, that of administration per oral and absorption via the gastrointestinal tract.

### BACKGROUND OF THE INVENTION

Therapeutically efficient administration of insulin in the treatment of diabetes type 1 and 2 is traditionally made via the subcutaneous (sc) route of administration which requires frequent injections. To decrease the discomfort that injections might give, several alternative non-invasive routes of administration have been investigated in the past. However, such alternatives to subcutaneous administration of insulin have only very recently become available. These are limited to inhaled insulin (systemic delivery of insulin via the pulmonary route of administration made by inhalation of a spray dried powder) and to buccal insulin delivery (systemic insulin delivery via the absorption of insulin in the oral cavity) made by spraying a liquid insulin composition directly into the oral cavity. The commercially available products of these alternative insulin administration forms are registered under the trade names Exubera® and Ora-Lyn®, respectively

In particular, the oral route has attracted considerable interest. The latter efforts are driven by, among other factors, that the oral route of administration is the most convenient and most familiar route of administration. However, oral administration of insulin is associated with several major barriers that remain to be overcome.

Normal therapeutically useful insulin solutions made for subcutaneous injection do not exceed concentrations of 100 U/ml or 3.5 mg/ml. But for specific purposes like insulin solutions for infusion pumps (human semisynthetic insulin, Genapol stabilized, 400 U/ml by Hoechst) and concentrated insulin solutions intended for inhalation (AerX insulin blister 1500 U/ml; Diabetes Tech Therapeut (2002), vol. 4, page 499), concentrated insulin solutions have been described both at acidic conditions and at neutral pH in the presence of phenol and zinc or a combination thereof (EP 1 117 114 A2 and references cited therein).

Highly concentrated solutions of porcine insulin at acidic pH are described in Diabetes Care (1981), volume 4, page 266. However, the insulin in the composition is not stable due to deamidation and polymerisation. This together with the acidic pH, renders the composition less suitable for further compounding and composition so as to develop suitable dosage forms for alternative routes of administration including but not limited to the oral route of administration.

At neutral pH, the physical stability of the native (i.e. human or animal) insulin is increased by the presence of 2-4 zinc ions per insulin hexamer, 0.1-0.5 % w/v phenol and 5-150 mM sodium chloride. Stable porcine insulin solutions comprising zinc at neutral pH have been made with concentrations up to 6 mM porcine insulin (Brange and Havelund in Artificial Systems for Insulin Delivery, Brunetti et al. eds. Raven Press 1983).

Stable highly concentrated insulin solutions which comprise no or only small amounts of one or more of these additives, in particular stable highly concentrated insulin solutions comprising no or only small amounts of zinc ions and/or phenol, would be particular useful for subsequent compounding for delivery of insulin via alternative routes such as the gastrointestinal tract. Thus there is a need of highly concentrated insulin solutions with pH in the range of 6.5 - 9.0 and comprising no or only small amounts of zinc and/or phenol.

### SUMMARY OF THE INVENTION

One object of the invention is to provide an aqueous solution comprising an insulin, less than 2 zinc ions per insulin hexamer and 60 mM or less phenolic components, wherein the insulin concentration is above 12 mM.

In one aspect the aqueous solution according to the invention has a pH in the range from 6.5 to 9.0.

A pharmaceutical composition comprising an insulin, less than 2 zinc ions per insulin hexamer and 60 mM or less phenolic components, and optionally one or more excipients is also obtained by the invention.

In one aspect a pharmaceutical composition is obtained according to the invention wherein the concentration of the insulin is larger than 12 mM.

Furthermore a method for preparing an aqueous insulin solution comprising less than 2 zinc ions per insulin hexamer and 60 mM or less phenolic components, wherein the insulin concentration is above 12 mM, is obtained.

### DESCRIPTION OF THE DRAWINGS

**Figure 1**
   Blood glucose reduction after application of stable and highly concentrated zinc- and phenol-free Insulin Aspart® solution into duodenum of fasted SPRD rats (n=2) by direct injection administration.
**Figure 2**
   Blood glucose reduction after application of stable and highly concentrated zinc- and phenol-free Insulin Aspart® in poloxamer surfactant mixture into ileum of fasted SPRD rats (n=3) by direct injection administration.
**Figure 3**
   Blood glucose reduction after application of stable and highly concentrated zinc- and phenol-free Insulin Aspart® micoremulsions into ileum of fasted SPRD rats (n=2-4) by direct injection administration.

### DESCRIPTION OF THE INVENTION

The present invention relates to insulin aqueous solutions which enable unique insulin pharmaceutical compositions to be made which in turn are useful for the compounding of alternative insulin administration forms such as insulin compositions intended for oral administration.

Freeze-dried powders or particles obtained from highly concentrated solutions are generally more homogenous than powders or particles from freeze-dried suspensions.

It is known that the thermodynamic driving force of drug absorption is based on among other things the concentration gradient between the drug dissolved in proximity of its site of absorption and the blood.

Insulins may be directly dissolved to obtain acidic aqueous insulin solutions, however it would be advantageous to dissolve insulins in non-acidic aqueous solutions to avoid fibrillation and chemical deteoriation of the insulin molecule in the solution.

To obtain stabile non-acidic insulin solutions, 2 or more zinc ions per insulin hexamer have previously been added to the insulin solution. Alternatively, as known from the insulin composition Apidra, detergents have been added to obtain the same stability effect. For some insulin derivatives, such as Detemir, the addition of zinc has also increased the solubility of insulin.

One objective of the invention is to provide highly concentrated non-acidic insulin solutions and compositions, methods of their preparation, and uses thereof. The invention thus provides highly concentrated insulin aqueous solutions suited for development of dosage forms suitable for several alternative routes of administration including, but not limited to, that of administration per oral and absorption via the gastrointestinal tract.

The inventors have surprisingly found that stable insulin solutions can be prepared with exceptionally high insulin concentration.

The following is a non-limiting list of aspects, which is further described elsewhere herein:
In aspect 1 of the invention an aqueous solution is obtained comprising an insulin, less than 2 zinc ions per insulin hexamer and 60 mM or less phenol and/or m-cresol, wherein the insulin concentration is above 12 mM.
Aspect 2. An aqueous solution according to aspect 1 wherein the insulin concentration is above 15 mM.
Aspect 3. An aqueous solution according to aspect 2 wherein the insulin concentration is above 20 mM.
Aspect 4. An aqueous solution according to aspect 3 wherein the insulin concentration is above 25 mM.
Aspect 5. An aqueous solution according to aspect 4 wherein the insulin concentration is above 30 mM.
Aspect 6. An aqueous solution according to aspect 1 wherein the insulin concentration is in the range from 12 mM - 60 mM.
Aspect 7. An aqueous solution according to aspect 6 wherein the insulin concentration is in the range from 12 mM - 55 mM.
Aspect 8. An aqueous solution according to aspect 7 wherein the insulin concentration is in the range from 12 mM - 50 mM.
Aspect 9. An aqueous solution according to aspect 8 wherein the insulin concentration is in the range from 15 mM - 50 mM.
Aspect 10. An aqueous solution according to aspect 9 wherein the insulin concentration is in the range from 20 mM - 50 mM.
Aspect 11. An aqueous solution according to aspect 6 wherein the insulin concentration is in the range from 25 mM - 60 mM.
Aspect 12. An aqueous solution according to aspect 11 wherein the insulin concentration is in the range from 25 mM - 50 mM.
Aspect 13. An aqueous solution according to aspect 11 wherein the insulin concentration is in the range from 30 mM - 60 mM.
Aspect 14. An aqueous solution according to aspect 13 wherein the insulin concentration is in the range from 30 mM - 50 mM.
Aspect 15. An aqueous solution according to any one of the preceding aspects wherein the zinc content is 1 or less zinc ions per insulin hexamer
Aspect 16. An aqueous solution according to aspect 15, which is essentially free from zinc.
Aspect 17. An aqueous solution according to any one of the preceding aspects wherein the concentration of phenolic components is below 60 mM.
Aspect 18. An aqueous solution according aspect 17 wherein the concentration of phenolic components is below 40 mM.
Aspect 19. An aqueous solution according to aspect 18 wherein the concentration of phenolic components is below 20 mM.
Aspect 20. An aqueous solution according to aspect 19 which is essentially free from phenolic components.
Aspect 21. An aqueous solution according to any one of the preceding aspects which is essentially free from zinc and essentially free from phenolic components.
Aspect 22. An aqueous solution according to any one of the preceding aspects wherein the pH is in the range from 6.5 to 9.0.
Aspect 23. An aqueous solution according to aspect 22 wherein the pH is in the range from 6.5 - 8.5.
Aspect 24. An aqueous solution according to aspect 23 wherein the pH is in the range from 7.0 - 8.5.
Aspect 25. An aqueous solution according to any one of the preceding aspects wherein the salt concentration is below 0.5 M.
Aspect 26. An aqueous solution according to aspect 25 wherein the salt concentration is below 0.3 M.
Aspect 27. An aqueous solution according to aspect 26 wherein the salt concentration is below 0.15 M.
Aspect 28. An aqueous solution according to aspect 27 which is essentially free from salt.
Aspect 29. An aqueous solution according to any one of the preceding aspects which is essentially free from sodium chloride.
Aspect 30. An aqueous solution according to any one of the preceding aspects wherein the insulin is selected from the group consisting of human insulin, an insulin analogue and an insulin derivative.
Aspect 31. An aqueous solution according to aspect 30 wherein the insulin is selected from the group consisting of human insulin and an insulin analogue.
Aspect 32. An aqueous solution according to aspect 31 wherein the insulin is selected from the group consisting of:
   human insulin;
   an insulin analogue wherein the amino acid residue in position B28 of insulin is Pro, Asp, Lys, Leu, Val, or Ala and the amino acid residue in position B29 is Lys or Pro and optionally the amino acid residue in position B30 is deleted;
   des(B28-B30) human insulin, des(B27) human insulin or des(B30) human insulin;
   an insulin analogue wherein the amino acid residue in position B3 is Lys and the amino acid residue in position B29 is Glu or Asp;
   an insulin analogue wherein the amino acid in position A14 is selected from the group consisting of Lys, Glu, Arg, Asp, Pro and His, the amino acid in position B25 is His and which optionally further comprises one or more additional mutations;
   an insulin analogue wherein
      - the amino acid in position A8 is His and/or the amino acid in position A12 is Glu or Asp and/or the amino acid in position A13 is His, Asn, Glu or Asp and/or the amino acid in position A14 is Asn, Gln, Glu, Arg, Asp, Gly or His and/or the amino acid in position A15 is Glu or Asp; and
      - the amino acid in position B1 is Glu and/or the amino acid in position B16 is Glu or His and or the amino acid in position B25 is His and/or the amino acid in position B26 is His, Gly, Asp or Thr and/or the amino acid in position B27 is His, Glu, Lys, Gly or Arg and/or the amino acid in position B28 is His, Gly or Asp; and which optionally further comprises one or more additional mutations; and
   an insulin analogue wherein the amino acid in position A14 is selected from the group consisting of Lys, Glu, Arg, Asp, Pro and His; and the B-chain of the insulin analogue comprises at least two mutations relative to the parent insulin, wherein two or more mutations are in the form of deletions of the amino acids in positions B27, B28, B29 and B30, or a combination of a deletion of the amino acid in position B30 and a substitution of an amino acid selected from the amino acid substitutions in position: B25 to His, B26 to Gly or Glu, B27 to Gly or Lys and B28 to Asp, His, Gly, Lys or Glu.
Aspect 33. An aqueous solution according to aspect 32 wherein the insulin is selected from the group consisting of:
   human insulin;
   DesB30 human insulin;
   AspB28 human insulin;
   AspB28,DesB30 human insulin;
   LysB3,GluB29 human insulin;
   LysB28,ProB29 human insulin;
   GluA14,HisB25 human insulin;
   HisA14,HisB25 human insulin;
   GluA14,HisB25,DesB30 human insulin;
   HisA14,HisB25,DesB30 human insulin;
   GluA14,HisB25,desB27,desB28,desB29,desB30 human insulin;
   GluA14,HisB25,GluB27,desB30 human insulin;
   GluA14,HisB16,HisB25,desB30 human insulin;
   HisA14,HisB16,HisB25,desB30 human insulin;
   HisA8,GluA14,HisB25,GluB27,desB30 human insulin;
   HisA8,GluA14,GluB1,GluB16,HisB25,GluB27,desB30 human insulin; and
   HisA8,GluA14,GluB16,HisB25,desB30 human insulin.
Aspect 34. An aqueous solution according to any one of the preceding aspects wherein the concentration of detergents is below 60 mM.
Aspect 35. An aqueous solution according aspect 34 wherein the concentration of detergents is below 40 mM.
Aspect 36. An aqueous solution according to aspect 35 wherein the concentration of detergents is below 20 mM.
Aspect 37. An aqueous solution according to aspect 36 which is essentially free from detergents.
Aspect 38. A pharmaceutical composition comprising an insulin, less than 2 zinc ions per insulin hexamer and 60 mM or less phenol and/or m-cresol, and optionally one or more excipients.
Aspect 39. A pharmaceutical composition according to aspect 38 wherein the concentration of the insulin is larger than 12 mM.
Aspect 40. A pharmaceutical composition according to aspect 39 wherein the insulin concentration is above 15 mM.
Aspect 41. A pharmaceutical composition according to aspect 40 wherein the insulin concentration is above 20 mM.
Aspect 42. A pharmaceutical composition according to aspect 41 wherein the insulin concentration is above 25 mM.
Aspect 43. A pharmaceutical composition according to aspect 42 wherein the insulin concentration is above 30 mM.
Aspect 44. A pharmaceutical composition according to aspect 39 wherein the insulin concentration is in the range from 12 mM - 60 mM.
Aspect 45. A pharmaceutical composition according to aspect 44 wherein the insulin concentration is in the range from 12 mM - 55 mM.
Aspect 46. A pharmaceutical composition according to aspect 45 wherein the insulin concentration is in the range from 12 mM - 50 mM.
Aspect 47. A pharmaceutical composition according to aspect 46 wherein the insulin concentration is in the range from 15 mM - 50 mM.
Aspect 48. A pharmaceutical composition according to aspect 47 wherein the insulin concentration is in the range from 20 mM - 50 mM.
Aspect 49. A pharmaceutical composition according to aspect 44 wherein the insulin concentration is in the range from 25 mM - 60 mM.
Aspect 50. A pharmaceutical composition according to aspect 49 wherein the insulin concentration is in the range from 25 mM - 50 mM.
Aspect 51. A pharmaceutical composition according to aspect 49 wherein the insulin concentration is in the range from 30 mM - 60 mM.
Aspect 52. A pharmaceutical composition according to aspect 51 wherein the insulin concentration is in the range from 30 mM - 50 mM.
Aspect 53. A pharmaceutical composition according to any one of aspects 38-52 wherein the zinc content is 1 or less zinc ions per insulin hexamer.
Aspect 54. A pharmaceutical composition according to aspect 53 which is essentially free from zinc.
Aspect 55. A pharmaceutical composition according to any one of aspects 38-54 wherein the concentration of phenolic components is below 60 mM.
Aspect 56. A pharmaceutical composition according to aspect 55 wherein the concentration of phenolic components is below 40 mM.
Aspect 57. A pharmaceutical composition according to aspect 56 wherein the concentration of phenolic components is below 20 mM.
Aspect 58. A pharmaceutical composition according to aspect 57 which is essentially free from phenolic components.
Aspect 59. A pharmaceutical composition according to any one of aspects 38-58 which is essentially free from zinc and essentially free from phenolic components.
Aspect 60. A pharmaceutical composition according to any one of aspects 38-59 wherein the pH is in the range from 6.5 to about 9.0.
Aspect 61. A pharmaceutical composition according to aspect 60 wherein the pH is in the range from 6.5 - 8.5.
Aspect 62. A pharmaceutical composition according to aspect 61 wherein the pH is in the range from 7.0 - 8.5
Aspect 63. A pharmaceutical composition according to any one of aspects 38-62 wherein the salt concentration is below 0.5 M.
Aspect 64. A pharmaceutical composition according to aspect 63 wherein the salt concentration is below 0.3 M.
Aspect 65. A pharmaceutical composition according to aspect 64 wherein the salt concentration is below 0.15 M.
Aspect 66. A pharmaceutical composition according to aspect 65 which is essentially free from salt.
Aspect 67. A pharmaceutical composition according to any one of aspects 38-66 wherein the insulin is selected from the group consisting of human insulin, an insulin analogue and an insulin derivative.
Aspect 68. A pharmaceutical composition according to aspect 67 wherein the insulin is selected from the group consisting of human insulin and an insulin analogue.
Aspect 69. A pharmaceutical composition according to aspect 68 wherein the insulin is selected from the group consisting of:
   human insulin;
   an insulin analogue wherein the amino acid residue in position B28 of insulin is Pro, Asp, Lys, Leu, Val, or Ala and the amino acid residue in position B29 is Lys or Pro and optionally the amino acid residue in position B30 is deleted;
   des(B28-B30) human insulin, des(B27) human insulin or des(B30) human insulin;
   an insulin analogue wherein the amino acid residue in position B3 is Lys and the amino acid residue in position B29 is Glu or Asp;
   an insulin analogue wherein the amino acid in position A14 is selected from the group consisting of Lys, Glu, Arg, Asp, Pro and His, the amino acid in position B25 is His and which optionally further comprises one or more additional mutations;
   an insulin analogue wherein
      - the amino acid in position A8 is His and/or the amino acid in position A12 is Glu or Asp and/or the amino acid in position A13 is His, Asn, Glu or Asp and/or the amino acid in position A14 is Asn, Gln, Glu, Arg, Asp, Gly or His and/or the amino acid in position A15 is Glu or Asp; and
      - the amino acid in position B1 is Glu and/or the amino acid in position B16 is Glu or His and or the amino acid in position B25 is His and/or the amino acid in position B26 is His, Gly, Asp or Thr and/or the amino acid in position B27 is His, Glu, Lys, Gly or Arg and/or the amino acid in position B28 is His, Gly or Asp; and which optionally further comprises one or more additional mutations; and
   an insulin analogue wherein the amino acid in position A14 is selected from the group consisting of Lys, Glu, Arg, Asp, Pro and His; and the B-chain of the insulin analogue comprises at least two mutations relative to the parent insulin, wherein two or more mutations are in the form of deletions of the amino acids in positions B27, B28, B29 and B30, or a combination of a deletion of the amino acid in position B30 and a substitution of an amino acid selected from the amino acid substitutions in position: B25 to His, B26 to Gly or Glu, B27 to Gly or Lys and B28 to Asp, His, Gly, Lys or Glu.
Aspect 70. A pharmaceutical composition according to aspect 69 wherein the insulin is selected from the group consisting of:
   human insulin;
   DesB30 human insulin;
   AspB28 human insulin;
   AspB28,DesB30 human insulin;
   LysB3,GluB29 human insulin;
   LysB28,ProB29 human insulin;
   GluA14,HisB25 human insulin;
   HisA14,HisB25 human insulin;
   GluA14,HisB25,DesB30 human insulin;
   HisA14,HisB25,DesB30 human insulin;
   GluA14,HisB25,desB27,desB28,desB29,desB30 human insulin;
   GluA14,HisB25,GluB27,desB30 human insulin;
   GluA14,HisB16,HisB25,desB30 human insulin;
   HisA14,HisB16,HisB25,desB30 human insulin;
   HisA8,GluA14,HisB25,GluB27,desB30 human insulin;
   HisA8,GluA14,GluB1,GluB16,HisB25,GluB27,desB30 human insulin; and
   HisA8,GluA14,GluB16,HisB25,desB30 human insulin.
Aspect 71. A pharmaceutical composition according any one of aspects 38-70 wherein the concentration of detergents is below 40 mM.
Aspect 72. A pharmaceutical composition according to aspect 71 wherein the concentration of detergents is below 20 mM.
Aspect 73. A pharmaceutical composition according to aspect 72 which is essentially free from detergents.
Aspect 74. A method for preparing an aqueous insulin solution wherein the insulin concentration is above 12 mM, alternatively above 15 mM, 20 mM, 25 mM, 30 mM or 35 mM, comprising:
   a) Suspending or dissolving in H₂O an insulin or crystals of an insulin, which insulin comprises less than 2 zinc ions per insulin hexamer,
   b) measuring pH,
   c) if pH is below pH 6.5 or above pH 9.0, adjusting pH to pH 6.5 - 9.0, and
   d) optionally freeze-drying or spray-drying the insulin and subsequently redissolving the insulin in H₂O
      to obtain an aqueous insulin solution comprising an insulin, which insulin comprises less than 2 zinc ions per insulin hexamer, wherein the insulin concentration is above 12 mM, alternatively above 15 mM, 20 mM, 25 mM, 30 mM or 35 mM, and pH is 6.5 - 9.0.
Aspect 75. A method for preparing an aqueous insulin solution wherein the insulin concentration is above 12 mM, alternatively above 15 mM, 20 mM, 25 mM, 30 mM or 35 mM, comprising: Suspending or dissolving in H₂O an insulin or crystals of an insulin, which insulin comprises less than 2 zinc ions per insulin hexamer, measuring pH and optionally, when pH is below pH 6.5 or above pH 9.0, adjusting pH to pH 6.5 - 9.0, and further optionally freeze-drying or spray-drying the insulin and subsequently redissolving the insulin in H₂O, to obtain an aqueous insulin solution comprising an insulin, which insulin comprises less than 2 zinc ions per insulin hexamer, wherein the insulin concentration is above 12 mM, alternatively above 15 mM, 20 mM, 25 mM, 30 mM or 35 mM, and pH is 6.5 - 9.0.
Aspect 76. A method for preparing an aqueous insulin solution wherein the insulin concentration is above 12 mM, alternatively above 15 mM, 20 mM, 25 mM, 30 mM or 35 mM, according to aspect 74 or 75 comprising:
   a. Suspending a insulin, which insulin comprises less than 2 zinc ions per insulin hexamer, alternatively insulin, which insulin comprises less than 1 zinc ion per insulin hexamer, alternatively essentially zinc free insulin in H₂O to obtain a suspension wherein the insulin concentration is above 12 mM, alternatively above 15 mM, 20 mM, 25 mM, 30 mM or 35 mM, and
   b. Adjusting the pH to pH 6.5 - 9.0 whereby a solution is obtained.
Aspect 77. A method for preparing an aqueous insulin solution wherein the insulin concentration is above 12 mM, alternatively above 15 mM, 20 mM, 25 mM, 30 mM or 35 mM, according to aspect 74 or 75 comprising:
   a. Dissolving a sufficiently small amount of insulin, which insulin comprises less than 2 zinc ions per insulin hexamer, alternatively insulin, which insulin comprises less than 1 zinc ion per insulin hexamer, alternatively essentially zinc free insulin in H₂O to obtain a solution wherein the insulin concentration is below 12 mM,
   b. adjusting the pH to pH 6.5 - 9.0,
   c. freeze-drying or spray-drying the solution, and
   d. redissolving the freeze-dried product in H₂O without adjusting the pH
   to obtain a solution wherein the insulin concentration is above 12 mM, alternatively above 15 mM, 20 mM, 25 mM, 30 mM or 35 mM, and the pH is between 6.5 - 9.0.
Aspect 78. A method for preparing an aqueous insulin solution wherein the insulin concentration is above 12 mM, alternatively above 15 mM, 20 mM, 25 mM, 30 mM or 35 mM, according to aspect 74 or 75 comprising:
   a. Dissolving crystals of insulin, which insulin comprises less than 2 zinc ions per insulin hexamer, alternatively insulin, which insulin comprises less than 1 zinc ion per insulin hexamer, alternatively essentially zinc free insulin in H₂O to obtain a solution wherein the insulin concentration is above 12 mM, alternatively above 15 mM, 20 mM, 25 mM, 30 mM or 35 mM, and the pH is between 6.5 - 9.0.
Aspect 79. A method for treating a disease or disorder selected from the group consisting of hyperglycemia, type 2 diabetes, impaired glucose tolerance, type 1 diabetes, obesity, hypertension, syndrome X, dyslipidemia, cognitive disorders, atheroschlerosis, myocardial infarction, coronary heart disease and other cardiovascular disorders, CNS disorders such as Alzheimer's, stroke, inflammatory bowel syndrome, dyspepsia and gastric ulcers, said method comprising administering to a subject in need of such treatment an effective amount of the aqueous solution or pharmaceutical composition of any one of aspects 1-73.
Aspect 80. The aqueous solution or pharmaceutical composition according to any one of aspects 1-73 for use as a medicament.
Aspect 81. The aqueous solution or pharmaceutical composition according to any one of aspects 1-73 for use as a medicament for the treatment or prevention of hyperglycemia, type 2 diabetes, impaired glucose tolerance, type 1 diabetes, obesity, hypertension, syndrome X, dyslipidemia, cognitive disorders, atheroschlerosis, myocardial infarction, coronary heart disease and other cardiovascular disorders, CNS disorders such as Alzheimer's, stroke, inflammatory bowel syndrome, dyspepsia and gastric ulcers.
Aspect 82. The aqueous solution or pharmaceutical composition according to any one of aspects 1-73 for use as a medicament for delaying or preventing disease progression in type 2 diabetes.

By "an insulin" as used herein is meant human insulin, an insulin analogue or an insulin derivative.

By "insulin analogue" as used herein is meant a polypeptide having a molecular structure which formally can be derived from the structure of human insulin. Insulins from animals thus become analogues of human insulin. The structure of an analogue can be derived e.g. by deleting and/or substituting at least one amino acid residue occurring in the natural insulin and/or by inserting and/or adding at least one amino acid residue. The inserted, added and/or substituted amino acid residues will typically be codable amino acid residues.

The insulin analogues according to the present invention may be human insulin or an analogue thereof comprising one or more mutations in the A-chain and/ or the B-chain of the insulin.

In one embodiment an insulin analogue according to the invention comprises less than 8 modifications (substitutions, deletions, additions) relative to the parent insulin. In one embodiment an insulin analogue comprises less than 7 modifications (substitutions, deletions, additions) relative to the parent insulin. In one embodiment an insulin analogue comprises less than 6 modifications (substitutions, deletions, additions) relative to the parent insulin. In another embodiment an insulin analogue comprises less than 5 modifications (substitutions, deletions, additions) relative to the parent insulin. In another embodiment an insulin analogue comprises less than 4 modifications (substitutions, deletions, additions) relative to the parent insulin. In another embodiment an insulin analogue comprises less than 3 modifications (substitutions, deletions, additions) relative to the parent insulin. In another embodiment an insulin analogue comprises less than 2 modifications (substitutions, deletions, additions) relative to the parent insulin.

Examples of insulin analogues are such wherein Pro in position 28 of the B chain is mutated with Asp, Lys, Leu, Val, or Ala. In another aspect Lys at position B29 is mutated with Pro, Glu or Asp. Furthermore, Asn at position B3 may be mutated with Thr, Lys, Gin, Glu or Asp. Further examples of insulin analogues are the deletion analogues e.g. analogues where the B30 amino acid in human insulin has been deleted (des(B30) human insulin), insulin analogues wherein the B1 amino acid in human insulin has been deleted (des(B1) human insulin), des(B28-B30) human insulin and des(B27) human insulin. Insulin analogues comprising combinations of the above mutations are also examples of insulin analogues and insulin analogues wherein the A-chain and/or the B-chain have an N-terminal extension and insulin analogues wherein the A-chain and/or the B-chain have a C-terminal extension. Insulin analogues wherein the amino acid in position A14 is Glu or His, the amino acid in position B25 is His and which optionally further comprises one or more additional mutations are further examples of insulin analogues according to the invention.

By "insulin derivative" as used herein is meant a naturally occurring insulin or an insulin analogue which has been chemically modified, e.g. by guanylation, succinylation, acetylation or carbamoylation in one or more positions of the insulin backbone.

In one aspect an insulin of the invention is human insulin or an insulin analogue.

In a further aspect an insulin of the invention is selected from the group consisting of: human insulin; an insulin analogue wherein the amino acid residue in position B28 of insulin is Pro, Asp, Lys, Leu, Val, or Ala and the amino acid residue in position B29 is Lys or Pro and optionally the amino acid residue in position B30 is deleted; des(B28-B30) human insulin, des(B27) human insulin or des(B30) human insulin; an insulin analogue wherein the amino acid residue in position B3 is Lys and the amino acid residue in position B29 is Glu or Asp; and an insulin analogue wherein the amino acid in position A14 is Glu or His, the amino acid in position B25 is His and which optionally further comprises one or more additional mutations; an insulin analogue wherein
• the amino acid in position A8 is His and/or the amino acid in position A12 is Glu or Asp and/or the amino acid in position A13 is His, Asn, Glu or Asp and/or the amino acid in position A14 is Asn, Gln, Glu, Arg, Asp, Gly or His and/or the amino acid in position A15 is Glu or Asp; and
• the amino acid in position B1 is Glu and/or the amino acid in position B16 is Glu or His and or the amino acid in position B25 is His and/or the amino acid in position B26 is His, Gly, Asp or Thr and/or the amino acid in position B27 is His, Glu, Lys, Gly or Arg and/or the amino acid in position B28 is His, Gly or Asp; and
   which optionally further comprises one or more additional mutations; and
   an insulin analogue wherein the amino acid in position A14 is selected from the group consisting of Lys, Glu, Arg, Asp, Pro and His; and the B-chain of the insulin analogue comprises at least two mutations relative to the parent insulin, wherein two or more mutations are in the form of deletions of the amino acids in positions B27, B28, B29 and B30, or a combination of a deletion of the amino acid in position B30 and a substitution of an amino acid selected from the amino acid substitutions in position: B25 to His, B26 to Gly or Glu, B27 to Gly or Lys and B28 to Asp, His, Gly, Lys or Glu.

In a yet further aspect an insulin of the invention is selected from the group consisting of: human insulin; DesB30 human insulin; AspB28 human insulin; AspB28,DesB30 human insulin; LysB3,GluB29 human insulin; LysB28,ProB29 human insulin; GluA14,HisB25 human insulin; HisA14,HisB25 human insulin; GluA14,HisB25,DesB30 human insulin; HisA14, HisB25,DesB30 human insulin; GluA14,HisB25,desB27,desB28,desB29,desB30 human insulin; GluA14,HisB25,GluB27,desB30 human insulin; GluA14,HisB16,HisB25,desB30 human insulin; HisA14,HisB16,HisB25,desB30 human insulin; HisA8,GluA14,HisB25,GluB27,desB30 human insulin; HisA8,GluA14,GluB1,GluB16,HisB25,GluB27,desB30 human insulin; and HisA8,GluA14,GluB16,HisB25,desB30 human insulin.

A stable insulin solution or composition according to the invention is to be understood as a solution or composition wherein all components at room temperature are completely dissolved, i.e. there are no visible precipitates in the solution or composition.

An aqueous solution according to the invention has a concentration exceeding 12 mM. In one aspect the insulin concentration in the aqueous solution is above 15 mM, in another aspect the insulin concentration is above 20 mM, in a further aspect the insulin concentration is above 25 mM, in a further aspect the insulin concentration is above 30 mM, in a further aspect the insulin concentration is above 35 mM and in yet another aspect the insulin concentration is above 40 mM. In one aspect of the invention the insulin concentration is below 60 mM, in another aspect of the invention the insulin concentration is below 55 mM, in yet another aspect of the invention the insulin concentration is below 50 mM. In one aspect of the invention the insulin concentration is between 12 mM and 60 mM, in another aspect the insulin concentration in the solution is between 12 mM and 55 mM, in a further aspect the concentration is between 12 mM and 50 mM, in yet a further aspect the insulin concentration is between 15 mM and 50 mM and in still a further aspect the insulin concentration is between 20 mM and 50 mM.

A pharmaceutical composition according to the invention has a concentration exceeding 12 mM. In one aspect the insulin concentration in the pharmaceutical composition is above 15 mM, in another aspect the insulin concentration is above 20 mM, in a further aspect the insulin concentration is above 30 mM and in yet another aspect the insulin concentration is above 40 mM. In one aspect of the invention the insulin concentration is between 12 mM and 60 mM, in another aspect the insulin concentration in the solution is between 12 mM and 55 mM, in a further aspect the concentration is between 12 mM and 50 mM, in yet a further aspect the insulin concentration is between 15 mM and 50 mM and in still a further aspect the insulin concentration is between 20 mM and 50 mM.

An aqueous solution or pharmaceutical composition according to the invention may comprise a small amount of zinc and/or phenolic components.

Most commercial insulin and insulin analogue compositions currently marketed comprise zinc to enhance the stability of the insulin and to minimize the risk of fibrillation, i.e. the formation of fibrils. The amount of zinc in these compositions is normally 2, 3 or 4 zinc ions per insulin hexamer.

It has by the inventors been found that an aqueous solution or pharmaceutical composition comprising a high insulin concentration according to the invention may comprise only small amounts of or substantially no zinc.

In one aspect of the invention the aqueous solution or pharmaceutical composition comprises less than 2 zinc ions per insulin hexamer. In another aspect of the invention the aqueous solution or the pharmaceutical composition comprises 1 or less zinc ions per insulin hexamer. In yet a further aspect of the invention the aqueous solution or pharmaceutical composition is essentially free from zinc.

An aqueous solution or pharmaceutical composition of the invention may comprise small amounts of or no phenolic components. By the term "phenolic components" as used herein is meant phenol or phenol-like molecules such as e.g. m-cresol, m-paraben and phenol, which are often added to solutions and compositions to protect against microbial pollution.

In one aspect of the invention the aqueous solution or the pharmaceutical composition comprises 60 mM or less phenolic components. In another aspect an aqueous solution or a pharmaceutical composition of the invention comprises 40 mM or less phenolic components and in a further aspect an aqueous solution or a pharmaceutical composition of the invention comprises 20 mM or less phenolic components. In yet a further aspect an aqueous solution or a pharmaceutical composition of the invention is essentially free from phenolic components.

In one aspect of the invention the aqueous solution or the pharmaceutical composition comprises 60 mM or less phenol and/or m-cresol. In another aspect an aqueous solution or a pharmaceutical composition of the invention comprises 40 mM or less phenol and/or m-cresol and in a further aspect an aqueous solution or a pharmaceutical composition of the invention comprises 20 mM or less phenol and/or m-cresol. In yet a further aspect an aqueous solution or a pharmaceutical composition of the invention is essentially free from phenol and/or m-cresol.

In one aspect of the invention the aqueous solution or the pharmaceutical composition comprises 60 mM or less phenol. In another aspect an aqueous solution or a pharmaceutical composition of the invention comprises 40 mM or less phenol and in a further aspect an aqueous solution or a pharmaceutical composition of the invention comprises 20 mM or less phenol. In yet a further aspect an aqueous solution or a pharmaceutical composition of the invention is essentially free from phenol.

In one aspect an aqueous solution or a pharmaceutical composition of the invention is essentially free from zinc and phenolic components. In another aspect an aqueous solution or a pharmaceutical composition of the invention is essentially free from zinc and phenol.

In one aspect the salt concentration of an aqueous solution or a pharmaceutical composition according to the invention is below 0.5 M. In a further aspect the salt concentration of an aqueous solution or a pharmaceutical composition according to the invention is below 0.3 M. In a further aspect the salt concentration of an aqueous solution or a pharmaceutical composition according to the invention is below 0.15 M. In a yet further aspect an aqueous solution of the invention is essentially free from salt. In a still further aspect a pharmaceutical composition of the invention is essentially free from salt.

In this context the term "essentially free from" a given component shall mean a solution or composition comprising no or only trace amounts of the given component. It is known that trace amounts of e.g. zinc may be found in insulin solutions or compositions even without having been actively added thereto during any step of the process leading to the insulin solution or composition. In one aspect of the invention an aqueous solution or a pharmaceutical composition essentially free from zinc is understood as an aqueous solution or a pharmaceutical composition comprising trace amounts such as less than 0,1%, 0,05% or 0,03% of zinc. In a further aspect of the invention an aqueous solution or a pharmaceutical composition essentially free from zinc is understood as an aqueous solution or a pharmaceutical composition being free from zinc. In one aspect of the invention an aqueous solution or a pharmaceutical composition essentially free from phenol is understood as an aqueous solution or a pharmaceutical composition comprising trace amounts such as less than 0, 1 %, 0,05% or 0,03% of phenol. In a further aspect of the invention an aqueous solution or a pharmaceutical composition essentially free from phenol is understood as an aqueous solution or a pharmaceutical composition being free from phenol.

An aqueous solution or a pharmaceutical composition according to the invention may be in the pH range from about 6.5 to about 9.0. In one aspect the pH of an aqueous solution or a pharmaceutical composition of the invention is between 6.5 - 8.5, in a further aspect the pH of an aqueous solution or a pharmaceutical composition of the invention is between 7.0 - 8.5.

It has been found that highly concentrated neutral zinc- and phenol-free insulin aqueous solutions or compositions are reducing blood glucose levels to a great extend. In one aspect highly concentrated neutral zinc- and phenol-free insulin compositions of the invention are reducing blood glucose levels to a greater extent than unformulated highly concentrated insulin solutions. In a further aspect microemulsions comprising concentrated neutral zinc- and phenol-free insulin compositions of the invention are reducing blood glucose levels to a greater extent than unformulated highly concentrated insulin solutions.

Methods for the preparation of an aqueous insulin solution wherein the insulin concentration is above 12 mM, alternatively above 15 mM, 20 mM, 25 mM, 30 mM or 35 mM, are also covered by the invention.

Such methods are further described in the examples and may comprise suspending or dissolving in H₂O an insulin or crystals of an insulin, which insulin comprises less than 2 zinc ions per insulin hexamer, measuring pH and optionally adjusting pH if pH is below pH 6.5 or above pH 9.0, and further optionally freeze-drying or spray-drying the insulin and subsequently redissolving the insulin in H₂O, to obtain an aqueous insulin solution comprising an insulin, which insulin comprises less than 2 zinc ions per insulin hexamer, wherein the insulin concentration is above 12 mM, alternatively above 15 mM, 20 mM, 25 mM, 30 mM or 35 mM, and pH is 6.5 - 9.0.

In one aspect a method the preparation of an aqueous insulin solution according to the invention comprises suspending or dissolving a zinc-free insulin or crystals of a zinc-free insulin in H₂O, measuring pH and optionally adjusting pH, and further optionally freeze-drying or spray-drying the insulin and subsequently redissolving the insulin in H₂O, to obtain an aqueous insulin solution substantially free from zinc, wherein the insulin concentration is above 12 mM, alternatively above 15 mM, 20 mM, 25 mM, 30 mM or 35 mM, and pH is 6.5 - 9.0.

In one aspect a method for preparing an aqueous insulin solution comprising an insulin, less than 2 zinc ions per insulin hexamer and 60 mM or less phenolic components, wherein the insulin concentration is above 12 mM is obtained.

In one aspect a method for preparing an aqueous insulin solution comprising an insulin, less than 2 zinc ions per insulin hexamer and 60 mM or less phenolic components, wherein the insulin concentration is above 15 mM is obtained.

In one aspect a method for preparing an aqueous insulin solution comprising an insulin, less than 2 zinc ions per insulin hexamer and 60 mM or less phenolic components, wherein the insulin concentration is above 20 mM is obtained.

In one aspect a method for preparing an aqueous insulin solution comprising an insulin, less than 2 zinc ions per insulin hexamer and 60 mM or less phenolic components, wherein the insulin concentration is above 25 mM is obtained.

In one aspect a method for preparing an aqueous insulin solution comprising an insulin, less than 2 zinc ions per insulin hexamer and 60 mM or less phenolic components, wherein the insulin concentration is above 30 mM is obtained.

In one aspect a method for preparing an aqueous insulin solution comprising an insulin, less than 2 zinc ions per insulin hexamer and 60 mM or less phenolic components, wherein the insulin concentration is above 35 mM is obtained.

In another aspect a method for preparing an aqueous insulin solution comprising an insulin, less than 2 zinc ions per insulin hexamer and 60 mM or less phenolic components, wherein the insulin concentration is above 12 mM, alternatively above 15 mM, 20 mM, 25 mM, 30 mM or 35 mM, is obtained comprising the steps of:
a. Suspending a zinc free insulin in H₂O to obtain a suspension wherein the insulin concentration is above 12 mM, and
b. Adjusting the pH to pH 6.5 - 9.0, whereby a solution is obtained.

In a further aspect a method for preparing an aqueous insulin solution comprising an insulin, less than 2 zinc ions per insulin hexamer and 60 mM or less phenolic components, wherein the insulin concentration is above 12 mM, alternatively above 15 mM, 20 mM, 25 mM, 30mM or 35 mM, is obtained comprising the steps of:
a. Dissolving a sufficiently small amount of zinc free insulin in H₂O to obtain a solution wherein the insulin concentration is below 12 mM,
b. adjusting the pH to pH 6.5 - 9.0,
c. freeze-drying or spray-drying the solution, and
d. redissolving the freeze-dried product in H₂O without adjusting the pH to obtain a solution wherein the insulin concentration is above 12 mM, alternatively above 15 mM, 20 mM, 25 mM, 30 mM or 35 mM.

In a yet further aspect a method for preparing an aqueous insulin solution comprising an insulin, less than 2 zinc ions per insulin hexamer and 60mM or less phenolic components, wherein the insulin concentration is above 12 mM, alternatively above 15 mM, 20 mM, 25 mM, 30 mM or 35 mM, is obtained comprising the steps of:
a. Dissolving crystals of zinc free insulin in H₂O to obtain a solution wherein the insulin concentration is above 12 mM, alternatively above 15 mM, 20 mM, 25 mM, 30 mM or 35 mM, and the pH is pH 6.5 - 9.0.

The use of a solution comprising an insulin, less than 2 zinc ions per insulin hexamer and 60mM or less phenolic components, wherein the insulin concentration is above 12 mM, alternatively above 15 mM, 20 mM, 25 mM, 30 mM or 35 mM, for preparing a composition suitable for transmucosal delivery is also covered by the invention. Said composition enable sufficient solution concentration of insulin at pH 6.5 - 9.0 for uptake of insulin via the transmucosal route while comprising less than 2 zinc ions per insulin hexamer and 60mM or less phenolic components.

### PHARMACEUTICAL COMPOSITIONS

A pharmaceutical composition according to the invention is a composition comprising an insulin, less than 2 zinc ions per insulin hexamer, 60mM or less phenolic components and optionally one or more excipients, wherein the composition is prepared from an insulin solution wherein the insulin concentration is above 12 mM, alternatively above 15 mM, 20 mM, 25 mM, 30 mM or 35 mM. In one aspect a pharmaceutical composition according to the invention is a composition comprising an insulin, less than 2 zinc ions per insulin hexamer, 60mM or less phenolic components and optionally one or more excipients, wherein the insulin concentration is above 12 mM, alternatively above 15 mM, 20 mM, 25 mM, 30 mM or 35 mM.

Another object of the present invention is to provide a pharmaceutical composition comprising an insulin prepared from an insulin solution, in which solution the insulin concentration is above 12 mM, alternatively above 15 mM, 20 mM, 25 mM, 30 mM or 35 mM, according to the present invention, wherein said composition has a pH from 6.5 to 9.0. The composition may further comprise protease inhibitor(s) known to the person skilled in the art, a buffer system, preservative(s), tonicity agent(s), chelating agent(s), stabilizers and surfactants. In one aspect of the invention the pharmaceutical composition is an aqueous composition, i.e. composition comprising water. Such composition is typically a solution or a suspension. In a further aspect of the invention the pharmaceutical composition is an aqueous solution. The term "aqueous composition" is defined as a composition comprising at least 50 % w/w water. Likewise, the term "aqueous solution" is defined as a solution comprising at least 50 % w/w water, and the term "aqueous suspension" is defined as a suspension comprising at least 50 % w/w water.

In another aspect the pharmaceutical composition is a freeze-dried composition, whereto the physician or the patient adds solvents and/or diluents prior to use.

In another aspect the pharmaceutical composition is a dried composition (e.g. freeze-dried or spray-dried) ready for use without any prior dissolution.

In a further aspect the invention relates to a pharmaceutical composition comprising an aqueous solution or suspension of an insulin of the invention and a buffer, wherein said insulin is prepared from a solution of insulin present in a concentration from 12 mM or above, alternatively above 15 mM, 20 mM, 25 mM, 30 mM or 35 mM, and wherein said composition has a pH from 6.5 to 9.0.

Compositions intended for oral use may be prepared according to any known method, and such compositions may contain one or more agents selected from the group consisting of sweetening agents, flavouring agents, colouring agents, and preserving agents in order to provide pharmaceutically elegant and palatable preparations. Tablets may contain the active ingredient in a mixture with non-toxic pharmaceutically-acceptable excipients which are suitable for the manufacture of tablets. The tablets may be uncoated or they may be coated by known techniques to delay disintegration or release of the therapeutically active polypeptide.

The orally administerable compositions of the present invention may be prepared and administered according to methods well known in pharmaceutical chemistry, see Remington's Pharmaceutical Sciences, 17th ed. (A. Osol ed., 1985).

In one aspect of the invention, the pharmaceutical compositions of the present invention may be administered by means of solid dosage forms such as tablets and capsules. The tablets may be prepared by wet granulation, by dry granulation, by direct compression or melt granulation. Tablets for this invention may be prepared utilizing conventional tabletting techniques.

Compositions for oral use may also be presented as hard or soft gelatine capsules where the active ingredient is mixed with an inert solid diluent, for example, such as mannitol, maltodextrin, calcium carbonate, sodium carbonate, lactose, kaolin, calcium phosphate or sodium phosphate, or a soft gelatine capsule wherein the active ingredient is mixed with water or an oil medium, for example peanut oil, liquid paraffin, or olive oil.

Capsules for this invention may be prepared utilizing conventional methods. A general method of manufacture involves blending a therapeutically active polypeptide, alginate, a water-soluble diluent, a hydrophilic binder, and optionally a portion of a disintegrant. This blend is then granulated with an aqueous solution of the hydrophilic binder or an aqueous solution of the hydrophilic binder and surfactant in water, and milled, if necessary. The granules are dried and reduced to a suitable size. Any other ingredients, such as a lubricant, are added to the granules and mixed. The resulting mixture is then filled into a suitable size hard-shell gelatin capsule using conventional capsule-filling machines.

A composition of the invention may comprise further protease inhibitors such as EDTA (ethylenediamine tetraacetic acid) and benzamidine hydrochloride, but other commercially available protease inhibitors such as protease inhibitors of serine protease, aspartic proteases, cysteine proteases and metalloproteases may also be used.

Administration of pharmaceutical compositions according to the invention may be through the buccal, oral, ventricular and intestine routes of administration to patients in need of such a treatment.

Compositions of the current invention may be administered in several dosage forms, for example, as solutions, suspensions, emulsions, microemulsions, multiple emulsion, ointments, syrups, drops, gels, tablets, coated tablets, sublingual tablets, quick-dissolve buccal tablets, capsules, for example, hard gelatine capsules and soft gelatine capsules..

Compositions of the invention may further be compounded in, or attached to, for example through covalent, hydrophobic and electrostatic interactions, a drug carrier, drug delivery system and advanced drug delivery system in order to further enhance stability of the insulin compound, increase bioavailability, increase solubility, decrease adverse effects, achieve chronotherapy well known to those skilled in the art, and increase patient compliance or any combination thereof.

The term "stabilized composition" refers to a composition with increased physical stability, increased chemical stability or increased physical and chemical stability.

Aqueous suspensions may contain the insulin in admixture with excipients suitable for the manufacture of aqueous suspensions. Such excipients are suspending agents, dispersing or wetting agents. The aqueous suspensions may also contain one or more colouring agents, one or more flavouring agents, and one or more sweetening agents, such as sucrose or saccharin.

Oily suspensions may be formulated by suspending the active ingredient in a vegetable oil, for example arachis oil, olive oil, sesame oil or coconut oil, or in a mineral oil such as a liquid paraffin. The oily suspensions may contain a thickening agent, for example beeswax, hard paraffin or cetyl alcohol. Sweetening agents, and flavouring agents may be added to provide a palatable oral preparation. These compositions may be preserved by the addition of an anti-oxidant such as ascorbic acid.

Dispersible powders and granules suitable for preparation of an aqueous suspension by the addition of water provide the active compound in admixture with a dispersing or wetting agent, suspending agent and one or more preservatives. Additional excipients, for example, sweetening, flavouring, and colouring agents may also be present.

The pharmaceutical compositions comprising an insulin according to the present invention may also be in the form of oil-in-water emulsions. The oily phase may be a vegetable oil or a mineral oil or a mixture thereof. The emulsions may also contain suitable emulsifying agents, sweetening and flavouring agents.

Syrups and elixirs may be formulated with sweetening agents, for example glycerol, propylene glycol, sorbitol or sucrose. Such compositions may also contain a demulcent, preservative and flavouring and colouring agent.

In a further aspect of the invention, the composition further comprises a permeation enhancer. In general, permeation enhancers increase paracellular and trancellular transport of macromolecules by reversible altering the membrane integrity.

In a further aspect of the invention, the composition further comprises a mucoadhesive polymer. An intimate contact of the drug delivery system to the mucosa of the gastrointestinal tract can be obtained by use of such a mucoadhesive polymer. An intimate contact of the dosage form to the membrane seems advantageous as an enzymatic degradation of the therapeutically active polypeptide on the way between the delivery system and the absorption membrane can be avoided. Moreover, a step concentration gradient on the absorption membrane representing the driving force for passive drug uptake can be provided.

In a further aspect of the invention, the composition further comprises an inhibitor of a proteolytic enzyme(s) to further circumvent the enzymatic barrier and achieving the delivery of the therapeutically active polypeptide such as aminopeptidase inhibitor, amastatin, bestatin, boroleucine and puromycin. Examples of protease inhibitors are sodium glycolate, camostat mesilate, bacitracin, soybean trypsin inhibitor and aprotinin.

Entrapment and encapsulation is a technique used in drug delivery systems for therapeutically active polypeptides to optimize delivery properties including protection against enzymatic degradation. Entrapment or encapsulation could be in the form of polymeric drug delivery systems such as hydrogels and nanocapsules/microspheres, and lipid drug delivery systems such as liposomes and micro emulsions.

The solutions and compositions of this invention can be used in the treatment of various diseases.

In one aspect, the present invention relates to the use of a solution or composition according to the invention for the preparation of a medicament for the treatment of hyperglycemia, type 2 diabetes, impaired glucose tolerance, type 1 diabetes, obesity, hypertension, syndrome X, dyslipidemia, β-cell apoptosis, β-cell deficiency, myocardial infarction, inflammatory bowel syndrome, dyspepsia, cognitive disorders, e.g. cognitive enhancing, neuroprotection, atheroschlerosis, coronary heart disease and other cardiovascular disorders.

In another aspect of the invention a solution or composition according to the invention is used for the preparation of a medicament for delaying or preventing disease progression in type 2 diabetes.

The present invention is further illustrated by the following examples which, however, are not to be construed as limiting the scope of protection. The features disclosed in the foregoing description and in the following examples may, both separately and in any combination thereof, be material for realizing the invention in diverse forms thereof.

### THE FOLLOWING IS A NON-LIMITING LIST OF ASPECT FUTHER COMPRISED WITHIN

### THE SCOPE OF THE INVENTION:

1. An aqueous solution comprising an insulin, less than 2 zinc ions per insulin hexamer and 60 mM or less phenol and/or m-cresol, wherein the insulin concentration is above 12 mM.
2. An aqueous solution according to aspect 1 wherein the insulin concentration is above 20 mM.
3. An aqueous solution according to aspect 1 or 2, which is essentially free from zinc.
4. An aqueous solution according to any one of aspects 1-3 which is essentially free from phenolic components.
5. An aqueous solution according to any one of aspects 1-4 wherein the pH is in the range from 6.5 to 9.0.
6. An aqueous solution according to any one of aspects 1-5 wherein the salt concentration is below 0.15 M.
7. An aqueous solution according to anyone of aspects 1-6 which is essentially free from salt.
8. A pharmaceutical composition comprising an insulin, less than 2 zinc ions per insulin hex-amer and 60 mM or less phenolic components, and optionally one or more excipients.
9. A pharmaceutical composition according to aspect 8 wherein the concentration of the insulin is larger than 12 mM.
10. A method for preparing an aqueous insulin solution wherein the insulin concentration is above 12 mM, alternatively above 15 mM, 20 mM, 25 mM, 30 mM or 35 mM, comprising:
   a) Suspending or dissolving in H2O an insulin or crystals of an insulin, which insulin comprises less than 2 zinc ions per insulin hexamer,
   b) measuring pH,
   c) if pH is below pH 6.5 or above pH 9.0, adjusting pH to pH 6.5 - 9.0, and
   d) optionally freeze-drying or spray-drying the insulin and subsequently redissolving the insulin in H2O,
      to obtain an aqueous insulin solution comprising an insulin, which insulin comprises less than 2 zinc ions per insulin hexamer, wherein the insulin concentration is above 12 mM, alternatively above 15 mM, 20 mM, 25 mM, 30 mM or 35 mM, and pH is 6.5 - 9.0.
11. The aqueous solution or pharmaceutical composition according to any one of aspects 1-9 for use as a medicament.
12. An aqueous solution or pharmaceutical composition according to any one of aspects 1-9 for use in the preparation of a medicament for the treatment or prevention of hyperglycemia, type 2 diabetes, impaired glucose tolerance or type 1 diabetes,

### EXAMPLES

### Example 1 Preparation of a ∼16 mM solution of Insulin Aspart®

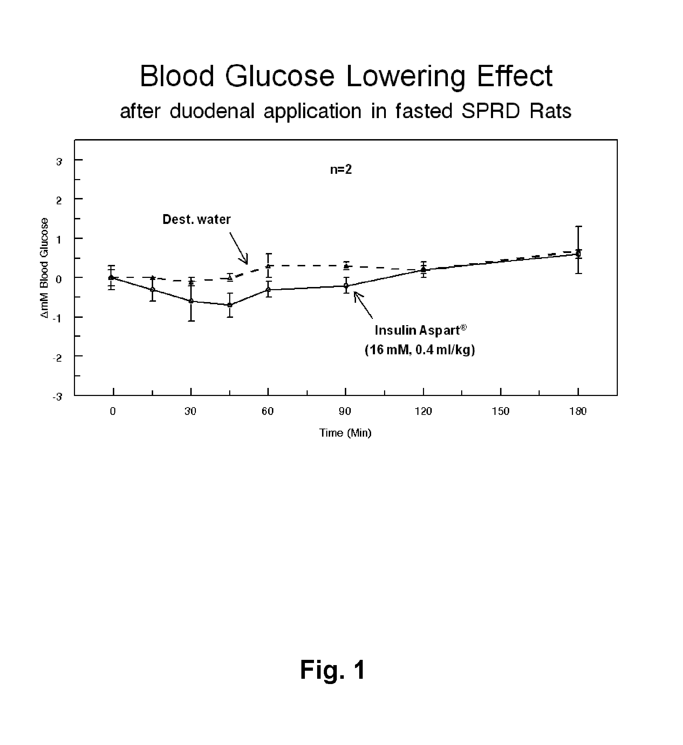

216 mg isoelectric precipitated Zn-free [B28-Asp] human insulin (Insulin Aspart®) was dispersed in 2 ml dest. water and the pH was cautiously adjusted to 7.4 with 2 N sodium hydroxide solution. The mixture was gently agitated on a roller mixer and left standing over night, resulting in a ∼16 mM solution of Insulin Aspart® (∼2700 IU/ml). Referred to as Solution 1. The solution was applied into duodenum of fasted SPRD rats (0.4 ml/kg, n=2) in comparison to destilled water and the resulting changes in blood glucose are shown in Figure 1.

### Example 2 Preparation of a 60 mM viscous solution of Insulin Aspart®

1 g isoelectric precipitated Zn-free [B28-Asp] human insulin (Insulin Aspart®) was dispersed in 100 ml water and the pH was adjusted to 7.4 with 2 N sodium hydroxide. The resulting solution was lyophilized.

720 mg of the lyophilized Insulin Aspart® was dispersed in 1.3 ml dest. water and the mixture was gently agitated on a roller-mixer, resulting in a 60 mM viscous solution of Insulin Aspart® (10000 IU/ml).

### Example 3 Preparation of a 36 mM solution of human insulin

420 mg crystallized Zn-free human insulin was dispersed in 1.6 ml dest. water and left with gentle agitation until complete dissolution. The volume was finally adjusted to 2.0 ml with water. This resulted in a 36 mM solution of human insulin (6000 IU/ml) with pH 8.

### Example 4 Preparation of surfactant based Compositions A-D by use of zinc- and phenol-free Insulin Aspart® Solution 1.

### Composition A: 3.7 mM insulin aspart (21.5 mg/ml)

A mixture of poloxamer 188, poloxamer 407 and insulin aspart was prepared in the following way: 1.065 ml insulin aspart solution (15.6 mM, prepared according to Example 1) and 3.435 ml MQ H₂O was added to a blend of 38.83 mg powder poloxamer 188 and 58.37 mg powder poloxamer 407. The mixture was left to mix by rotating on a rolling table. The resulting solution Composition A was perfectly clear and shown to be stable at ambient room temperature without any precipitation.

### Composition B: 5 mM insulin aspart (29 mg/ml)

A mixture of poloxamer 188, poloxamer 407 and insulin aspart was prepared in the following way: 1 ml insulin aspart 5.35 mM was added to a powder blend of 44.94 mg poloxamer 188 and 67.41 mg poloxamer 407. The mixture was left to mix by rotating on a rolling table. The resulting solution Composition B was perfectly clear and shown to be stable at ambient room temperature without any precipitation.

### Composition C: 10.3 mM insulin aspart (59.7 mg/ml)

A mixture of poloxamer 188, poloxamer 407 and insulin aspart was prepared in the following way: 0.5 ml insulin aspart Solution B1 and 0.375 ml MQ H₂O was added to a powder blend of 76.7 mg poloxamer 188 and 114.62 mg poloxamer 407. The mixture was left to mix by rotating on a rolling table. The resulting solution Composition C was perfectly clear and shown to be stable at ambient room temperature without any precipitation.

### Composition D: 18 mM insulin aspart (104.4 mg/ml)

A mixture of poloxamer 188, poloxamer 407 and insulin aspart was prepared in the following way: 2 ml insulin aspart 18 mM was added to a powder blend of 302.4 mg poloxamer 188 and 453.6 mg poloxamer 407. The mixture was left to mix by rotating on a rolling table. The resulting solution Composition D was perfectly clear and shown to be stable at ambient room temperature without any precipitation.

### Example 5 Preparation of highly concentrated zinc- and phenol-free insulin aspart containing microemulsions.

Any of the preceding examples may be used to achieve highly concentrated zinc-and phenol-free insulin containing microemulsions.

### Composition E:

As a matter of example neutral (pH 7.4) zinc- and phenol-free highly concentrated insulin containing microemulsions can be prepared based on the description in Example 1: a 14.8 mM stock solution was prepared by dispersing zinc- and phenol-free insulin in triethanolamine buffer and adjusting pH to 7.4 using NaOH.

Following the procedure described by Ritschel (W.A. Ritschel Microemulsions for Improved Absorption from the Gastrointestinal Tract. Meth Find Exp Clin Pharmacol 1991, 13: 205-220)] but with a somewhat different composition and the use of a highly concentrated zinc- and phenol-free neutral (pH 7.4) insulin aspart solution, a microemulsion containing 21.5 mg/ml insulin aspart was prepared by using:
1000 mg cetiol V
1647.05 mg labrasol
2352.95 mg plurol isostearique
1.667 ml of the 85.8 mg/ml zinc- and phenol-free insulin aspart solution (pH 7.4)

All components except insulin were mixed for 5 min at 40 °C followed by 10 min on a rolling table after which samples were put to the side for 30 min. The zinc- and phenol-free insulin aspart was then added and mixed in by gently turning and twisting the vial by hand over a period of 2 min.

### Example 6 Administration of Composition A to the gastrointestinal tract of rats

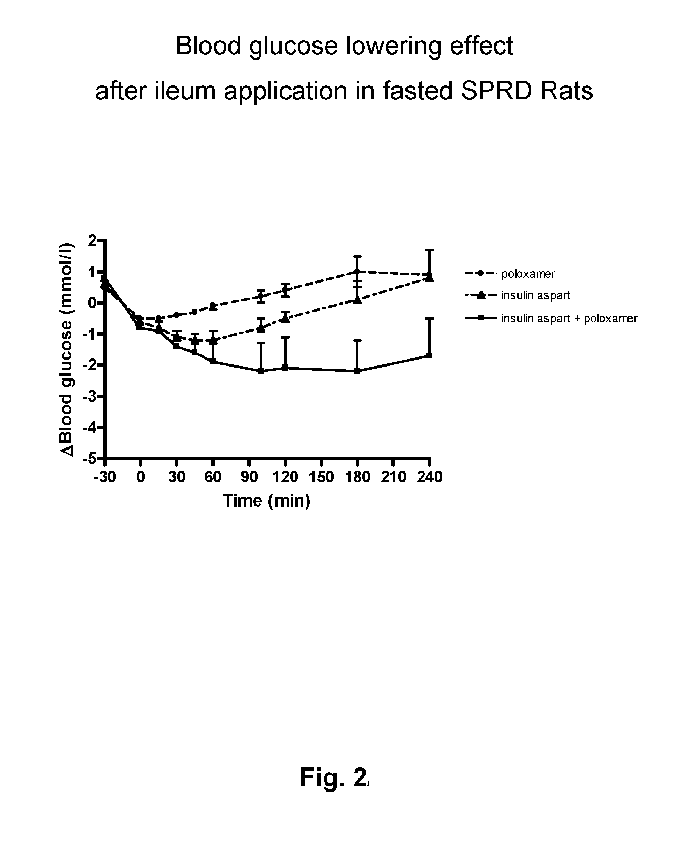

Composition A i.e., 21.5 mg/ml zinc- and phenol-free insulin aspart (pH 7.4) prepared according to Example 4 was administered in the ileum by direct injection to the intestine in non-diabetic SPRD rats and the blood glucose was monitored for 4 hours. The result is shown in Figure 2.

### Example 7 Administration of Composition E to the gastrointestinal tract of rats

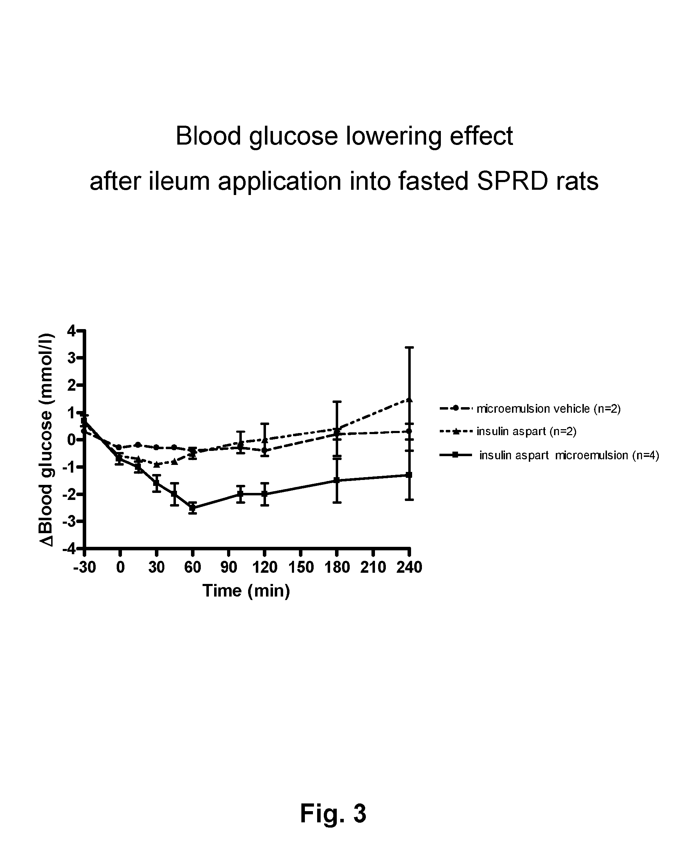

Composition E i.e., 21.5 mg/ml zinc- and phenol-free insulin aspart (pH 7.4) prepared according to Example 5 was administered in the ileum by direct injection to the intestine in non-diabetic SPRD rats and the blood glucose was monitored for 4 hours. The result is shown in Figure 3.

## Claims

1. An aqueous solution for preparing a composition suitable for transmucosal delivery, wherein the aqueous solutioncomprisesan insulin, less than 2 zinc ions per insulin hexamer and 60 mM or less phenol and/or m-cresol, wherein the insulin concentration is above 12 mM.

2. An aqueous solution according to claim 1 wherein the insulin concentration is above 20 mM.

3. An aqueous solution according to claim 1 or 2, which is essentially free from zinc.

4. An aqueous solution according to any one of claims 1-3 which is essentially free from phenolic components.

5. An aqueous solution according to any one of claims 1-4 wherein the pH is in the range from 6.5 to 9.0.

6. An aqueous solution according to any one of claims 1-5 wherein the salt concentration is below 0.15 M.

7. An aqueous solution according to anyone of claims 1-6 which is essentially free from salt.

8. A pharmaceutical composition suitable for transmucosal delivery comprising an insulin, less than 2 zinc ions per insulin hex-amer and 60 mM or less phenolic components, and optionally one or more excipients.

9. A pharmaceutical composition according to claim 8 wherein the concentration of the insulin is larger than 12 mM.

10. A method for preparing an aqueous insulin solution wherein the insulin concentration is above 12 mM, alternatively above 15 mM, 20 mM, 25 mM, 30 mM or 35 mM, comprising:
a) Suspending or dissolving in H2O an insulin or crystals of an insulin, which insulin comprises less than 2 zinc ions per insulin hexamer,
b) measuring pH,
c) if pH is below pH 6.5 or above pH 9.0, adjusting pH to pH 6.5 - 9.0, and
d) optionally freeze-drying or spray-drying the insulin and subsequently redissolving the insulin in H2O,
to obtain an aqueous insulin solution comprising an insulin, which insulin comprises less than 2 zinc ions per insulin hexamer, wherein the insulin concentration is above 12 mM, alternatively above 15 mM, 20 mM, 25 mM, 30 mM or 35 mM, and pH is 6.5 - 9.0.

11. The aqueous solution or pharmaceutical composition according to any one of claims 1-9 for use as a medicament.

12. An aqueous solution or pharmaceutical composition according to any one of claims 1-9 for use in the preparation of a medicament for the treatment or prevention of hyperglycemia, type 2 diabetes, impaired glucose tolerance or type 1 diabetes
